# EUROPEAN PATENT APPLICATION

(11) **EP 2 415 410 A1**
(43) Date of publication of application: **08.02.2012**
(21) Application number: 09842468.2
(22) Date of filing: 29.06.2009
(51) Int. Cl.: A61B 17/34

(54) **PUNCTURE NEEDLE FOR ORTHOPEDIC OPERATION**

(30) Priority: 03.04.2009 CN 200920020328 U
(71) Applicant: Shao, Weixing, Shandong 250014 (CN)
(72) Inventor: Shao, Weixing, Shandong 250014 (CN)
(74) Representative: Intès, Didier Gérard André
(86) International application number: PCT/CN2009/000723
(87) International publication number: WO 2010/111811

(57) **Abstract**

The present invention offers an embodiment of a medical surgery device, especially a puncture needle for bone surgery. The embodiment includes a needle tube and a needle tube seat for fastening the needle tube, and a needle core with cutting edge inserting into the needle tube and a needle core seat for fastening the needle core, and a hollow bolt through needle core is set on the needle tube seat, and in the embodiment, a nut matching with the hollow bolt is fastened on the said needle core seat, the nut and the needle core are coaxial. The said hollow bolt has a Luer conical fitting. The said bolt and the needle core and the needle core sear are one structure.

## Description

### Field of the Invention

The present invention relates to a kind of medical device, especially a puncture needle for bone surgery.

### Description of the Related Art

In existing technologies, the widely-known technology of puncture needle is in use now, the blade of the existing puncture needle is extruded out of the needle tube, it would get a big resistance when plugged into a bone, and made the needle core shrinked into the interior of the needle tube which brings inconvenience for surgery; and it's hard for the needle core to be removed after puncturing is finished because the high density bone clamps it, and the needle core can even be separated from the needle core seat, which causes unnecessary trouble for surgery, and can prevent it from proceeding smoothly. Those are the shortcomings of the existing technologies.

### Summary of the invention

The purpose of the present invention is to offer a technical embodiment of a puncture needle for bone surgery aiming at the shortcomings of existing technologies. In the embodiment, a nut matching with a hollow bolt is fastened on the needle core seat, the nut can be screwed into the hollow bolt with needle core to make the needle core seat and needle tube seat fixed, which is conducive for insertion of needle and withdraw of needle core.

The embodiment is achieved through the following technical measures: the puncture needle includes a needle tube and a needle tube seat for fastening the needle tube, a needle core with blade inserting into the needle tube and a needle core seat for fastening the needle core, and also a hollow bolt through the needle core on the needle tube seat, the features of the embodiment are that a nut matching with a hollow bolt is fastened on the said needle core seat, the nut and the said needle core are coaxial. The specific features of the embodiment also include that the said hollow bolt has a Luer conical fitting. The said nut and needle core and needle core seat are one structure.

The beneficial effects of the embodiment can be recognized by the above narration, in the embodiment, a nut matching with a hollow bolt is fastened on the said needle core seat, the nut and the said needle core are coaxial. In this structure, the nut on the needle core seat can be spun into the hollow bolt to make the needle core seat and needle tube seat fasten, when inserting into bone, the needle core seat and needle tube seat cannot be separated because of the connection between the nut and hollow bolt, it is conducive for the insertion of puncture needle; and it is easy for removing of the needle core, as long as rotating the needle core seat to separate the nut and the hollow bolt connected with thread, the operation is very convenient to ensure it is completed smoothly. Therefore, compared to the existing technologies, the present invention has substantive features and advantages, it is also obvious for its beneficial effects of implementation.

### Description of the drawings:

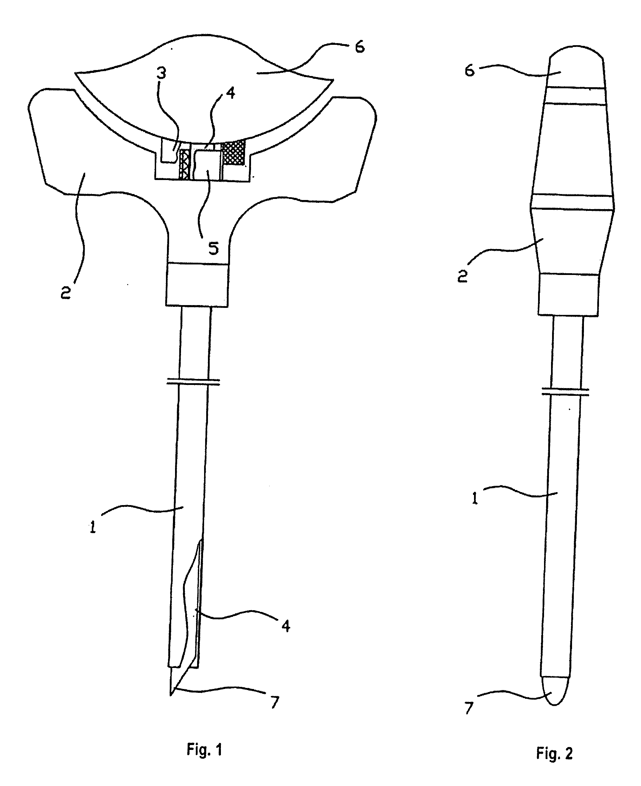

FIG. 1 is structure view of embodiment of the invention.
FIG. 2 is the left view of the FIG. 1.

Where, 1 is needle tube, 2 is needle tube seat, 3 is nut, 4 is needle core, 5 is hollow bolt, 6 is needle core seat, 7 is cutting edge.

### Detailed description of the invention

In order to clearly explain the technical features of the embodiment, the following will describe the embodiment by an embodiment and its drawings.

As it can be seen from the drawings, the puncture needle for bone surgery of the embodiment includes a needle tube 1 and a needle tube seat 2 for fastening the needle tube 1, and a needle core 4 with cutting edge inserting into the needle tube 1 and a needle core seat 6 for fastening the needle core 4, and a hollow bolt 5 through needle core 4 on the needle tube seat 2, the hollow bolt 5 has a Luer conical fitting. In the embodiment, a nut matching with the hollow bolt 5 is fastened on the said needle core seat 6, the nut 3 and the needle core 4 and needle core seat 6 are one structure made by injection molding, adhering or assembling. In addition, the said nut 3 and the said needle core 4 are coaxial.

## Claims

1. A puncture needle for bone surgery includes a needle tube and needle tube seat for fastening the needle tube, and a needle core with cutting edge inserting into the needle tube and a needle core seat for fastening the needle core, and a hollow bolt through needle core on the needle tube seat, wherein a nut matching with the hollow bolt is fastened on said needle core seat, the nut and the needle core being coaxial.

2. The puncture needle for bone surgery of claim 1, wherein the said hollow bolt has a Luer conical fitting.

3. The puncture needle for surgery of claim 1 or 2, wherein the said nut and the needle core and the needle core sear are one structure.
